(19) Europäisches Patentamt
European Patent Office
Office européen des brevets

(11) Publication number: 0 060 099 A1

(12) **EUROPEAN PATENT APPLICATION**

(21) Application number: 82301121.8

(22) Date of filing: 05.03.82

(51) Int. Cl.³: C 07 H 19/06
C 07 H 19/08, A 61 K 31/70

(30) Priority: 11.03.81 GB 8107629

(43) Date of publication of application: 15.09.82 Bulletin 82/37

(84) Designated Contracting States: CH DE FR GB IT LI NL

(71) Applicant: BEECHAM GROUP PLC
Beecham House Great West Road
Brentford Middlesex(GB)

(72) Inventor: Mock, Graham Andrew
34 Arundel Road Sands
High Wycombe Bucks(GB)

(72) Inventor: Harnden, Michael Raymond
47 GuildfordRoad
Horsham Sussex(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Antiviral agents, their preparation and use.**

(57) Compounds of formula (I)

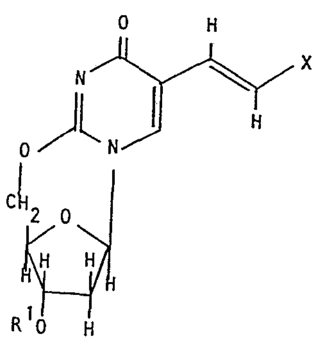

(I)

wherein X represents a halogen atom and
$R^1$ represents a hydrogen atom or a lower alkanoyl group.
possess antiviral activity, and are useful in the treatment of infections caused by herpes viruses.

EP 0 060 099 A1

Croydon Printing Company Ltd.

# ANTIVIRAL AGENTS,
# THEIR PREPARATION AND USE

This invention relates to certain deoxyuridine compounds which have antiviral activity.

British Patent specification No. 1,601,020 discloses 2'-deoxy-5-(2-halogenovinyl)-uridines, which are said to possess antiviral activity.

It has now been discovered that a class of novel 2 ,5'-anhydro nucleosides have antiviral activity.

According to the present invention, there is provided a compound of formula (I):

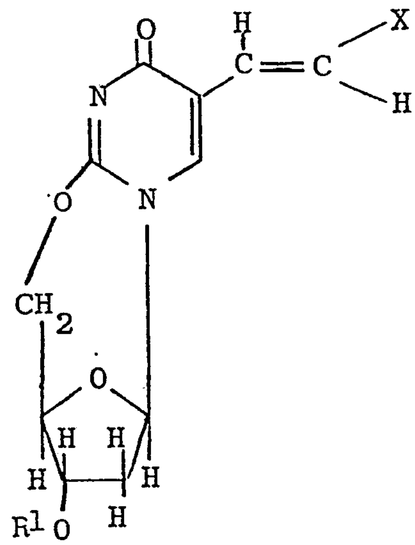

(I)

in which X represents a halogen atom, and

$R^1$ represents a hydrogen atom or a lower alkanoyl group.

Preferably, X represents a bromine atom and $R^1$ is acetyl.

When used herein, the term "lower" means that the group contains from 1 to 6 carbon atoms, preferably from 1 to 4 carbon atoms.

The compounds of the invention are particularly useful in the treatment of infections caused by herpes viruses, such as herpes simplex type 1, herpes simplex type 2 and varicella.

The compounds of formula (I) are also useful as intermediates in the synthesis of other biologically active 2'-deoxynucleosides.

The compounds of formula (I) may be prepared by reacting a compound of formula (II):

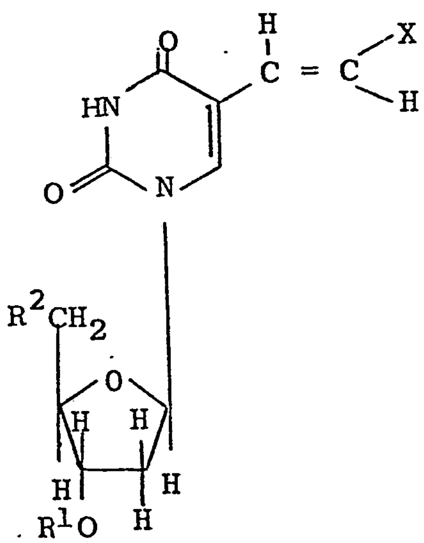

(II)

Wherein $R^1$ and X are as defined in formula (I) and $R^2$ is a leaving group, with a strong hindered base, such as; 1,5-diazabicyclo [4,3,0] non-5-ene (DBN), or 1,5-diazabicyclo [5,4,0] undec-7-ene (DBU).

preferably in an organic, preferably a polar, solvent. The preferred solvent is acetonitrile, and the reaction is suitably carried out under reflux. Preferably, $R^2$ is a tosyloxy, mesyloxy, p-bromotosyloxy group or a chlorine, bromine or iodine atom.

The product may be purified chromatographically, suitably by thin layer chromatography.

The compounds of formula (II) may themselves be made by reacting a deoxyuridine derivative of formula (III):

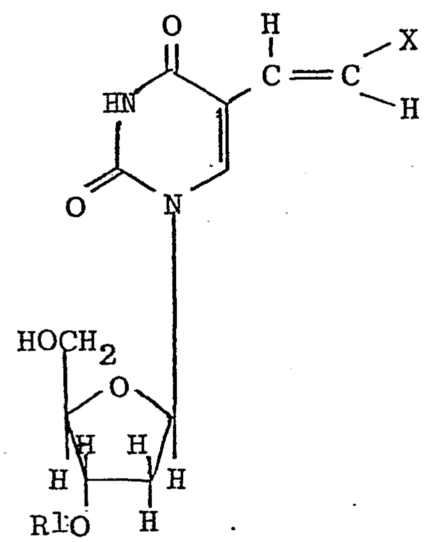

(III)

wherein $R^1$ represents a hydrogen atom or a lower alkanoyl group with a compound or mixture of compounds, which can replace the 5' hydroxyl group with the leaving group $R^2$.

Suitable compounds or mixtures for this replacement reaction will be known to those skilled in the art, but the following may be mentioned as preferred.

Sulphonyl halides (particularly the chloride), sulphonic acid anhydrides, and mixtures of triphenylphosphine and bromine or iodine. A particularly preferred compound is p-toluene sulphonyl chloride.

The reaction may take place in an organic, preferably a polar, solvent. A suitable solvent is pyridine, and the reaction is conveniently carried out at room temperature.

The compound of formula (II) may be purified chromatographically, suitable by column chromatography on silica gel.

The preparation of the compounds of formula (III) where $R^1$ is a lower alkanoyl group is described in our co-pending UK Patent applications, Nos. 8108755 and 8113618.

The compound of formula (I) may be utilised in the form of a pharmaceutical composition. Accordingly, in a further aspect of the invention, there is provided a pharmaceutical composition which comprises a compound of the formula (I) together with a pharmaceutically acceptable carrier or excipient.

Compositions which may be given by the oral route may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterils liquid carrier for injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books, and the British Pharmacopaeia.

The composition for application to the eyes may be a conventional eye-drop composition well known in the art.

Preferably the compositions of this invention are in unit dosage form or in some other form such that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound depends on the particular compound employed, but is in general in the range of from 1.0 mg/kg/day to 20 mg/kg of body weight per day or more usually 2.0 mg/kg/day to 10 mg/kg/day.

In a further aspect of the invention, there is provided a method of treating viral infections in a human or non-human animal, which comprises administering to the animal an effective, non-toxic amount of a compound of formula (I).

The following Examples illustrate the invention.

Example 1

(a) E-5-(2-Bromovinyl)-5'-O-p-toluenesulphonyl-2'-deoxyuridine

A solution of E-5-(2-bromovinyl)-2'-deoxyuridine (1 g, 3 mmol) and p-toluenesulphonyl chloride (0.63 g, 3.2 mmol) in dry pyridine (10 mL) was stirred at room temperature for 20 hours. A further quantity of p-toluenesulphonyl chloride (0.2 g, 1 mmol) was added and stirring was continued for 8 hours. The reaction mixture was poured into dichloromethane/water (100 mL/100 mL) and the aqueous layer was separated and further extracted with dichloromethane. The separate organic extracts were washed with saturated sodium bicarbonate solution (100 mL) and saturated sodium chloride solution (100 mL), and then combined and dried (sodium sulphate). Evaporation of the solvent under reduced pressure gave an oil (2 g) which was purified by column chromatography on silica gel (50 g). Elution with ethyl acetate yielded first the 3', 5'-ditosylate (0.415 g, 28%). Further elution with ethyl acetate afforded the 5'-monotosylate (0.54 g, 37%). A sample recrystallised from ethanol had mp 163-65°C;

$\nu$ max (KBr) 3420 (NH), 1714, 1700, 1665 (C=O) $cm^{-1}$;

$^1H$ nmr (80 MHz) ($CDCl_3$ + $(CD_3)_2SO$)

$\delta$ 2.23 (2H,m,2'-$CH_2$), 2.47 (3H,s,$CH_3$), 4.2 (4H,m,3'-CH, 4'-CH,5'-$CH_2$), 5.3 (1H,d,OH), 6.27 (1H,t,J=6.7Hz,1'-CH), 6.73 (1H,d,J=13.3Hz,CH=CHBr), 7.67 (6H,6 line multiplet, aromatic H's, 6-CH, CH=CHBr), 11.35 (1H,s,NH); m/e 488, 486 ($M^+$, 0.5%), 407 (1.5), 216 (9.3), 172 (20), 137 (100), 81 (97).

<u>Analysis</u>

Found: C, 44.74; H, 3.66; N, 5.54. $C_{18}H_{19}BrN_2O_7S$
requires: C, 44.36; H, 3.93; N, 5.75%

(b) 2,5'-Anhydro-E-5-(2-bromovinyl)-2'-deoxyuridine

A solution of E-5-(2-bromovinyl)-5'-O-p-toluenesulphonyl-2'-deoxyuridine (0.4 g, 0.82 mmol) and 1,5-diazabicyclo [4.3.0] non-5-ene (0.108 g, 0.92 mmol), in dry acetonitrile (10 mL) was heated under reflux for 4 hours (a white, crystalline solid was slowly precipitated over this period). The reaction mixture was filtered and the residue washed with acetonitrile (3 x 10 mL). The acetonitrile extracts were combined and concentrated under reduced pressure to give a yellow oil (0.4 g) which was purified by preparative thin layer chromatography. Elution with dichloromethane/methanol (85:15) gave the 2,5'-cyclonucleoside (0.123 g, 48%) as a white solid;

$^1H$ nmr (80 MHz) $[(CD_3)_2SO]$

$\delta$ 4.33 (4H,m,3',4'-CH,5'-$CH_2$), 5.33 (1H,m,OH),
6.17 (1H,dd,$J_{AX}$=6.7Hz,$J_{BX}$=2.6Hz,1'-CH),
6.83 (1H,d,J=13.3Hz,<u>CH</u>=CHBr), 7.57 (1H,d,J=13.3Hz,CH=<u>CH</u>Br),
8.13 (1H,s,6-CH);

m/e 315/317 ($M^+$, 2%), 261 (2), 158 (3), 98 (10), 52 (100).

Example 2

(a) 3'-O-Acetyl-E-5-(2-bromovinyl)-5'-O-p-toluenesulphonyl-2'-deoxyuridine

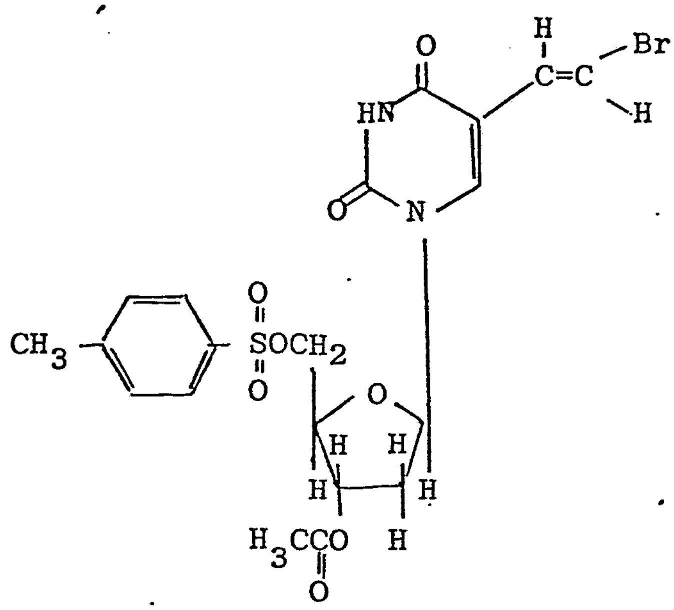

3'-O-Acetyl-E-5-(2-bromovinyl)-2'-deoxyuridine [a] (4.2g, 11.2 mmol) and p-toluenesulphonyl chloride (3.45 g, 18.1 mmol) were stirred in pyridine (50 mL) at 25$^o$ for 40 hours. The mixture was poured into water (400 mL) and extracted with chloroform (3 x 200 mL). The chloroform solution was washed with saturated sodium bicarbonate solution (2 x 150 mL) and water (200 mL) and dried (anhydrous sodium sulphate). The solvent was removed under reduced pressure to give an oil which was chromatographed on silica gel (eluting with chloroform/methanol (30:1)), to give a yellow foam. This was crystallised with chloroform/hexane and 3'-O-acetyl-E-5-(2-bromovinyl)-5'-O-p-toluenesulphonyl-2'-deoxyuridine was isolated as a white solid in 57% yield, with mp. 147-150$^oC$;

$\nu$ max (nujol) 3200, 1750, 1710, 1690, 1630, 1600 $cm^{-1}$;

$^1H$ nmr $[(CD_3)_2SO]$:

δ 2.02 (3H,s,$CH_3\underset{\overset{\|}{O}}{C}$), 2.38 (2H,m,2'$CH_2$), 2.4 (3H,s,<u>$CH_3$</u>-Ph), 4.14 (1H,m,4'CH), 4.28 (2H,m,5'$CH_2$), 5.13 (1H,m,3'CH), 6.1 (1H,t,J=7.5Hz,1'CH), 6.77 (1H,d,J=14Hz, <u>CH</u>=CHBr), 7.3 (1H,d,J=14Hz, CH=<u>CH</u>Br), 7.4 - 7.8 (4H,m,aromatic), 7.8 (1H,s,6-CH), 11.67 (1H,br.s,$D_2O$ exchangeable, NH).

<u>Analysis</u>

Found: C,45.23; H, 3.78; N, 5.41% $C_{20}H_{21}BrN_2O_8S$
requires: C, 45.38; H, 4.00; N,5.29%

[a]Described in U.K. Patent Application Nos. 8108755 and 8113618.

(b) <u>3'-O-Acetyl-2,5'-anhydro-E-5-(2-bromovinyl)-2'-deoxyuridine</u>

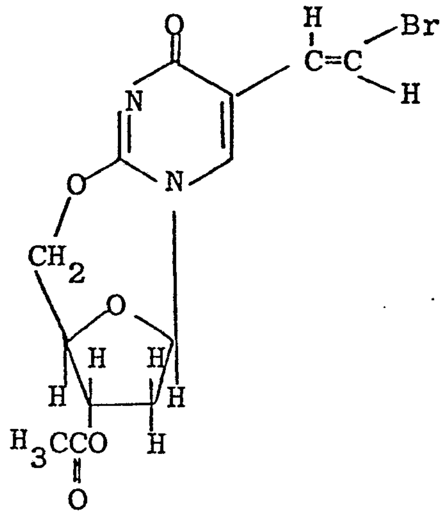

A solution of 3'-O-acetyl-E -5-(2-bromovinyl)-5'-O-p-toluenesulphonyl-2'-deoxyuridine (2.6 g, 4.9 mmol) and 1,5-diazabicyclo [4.3.0] non-5-ene (DBN) (0.67 g, 5.4 mmol) in dry acetonitrile (60 mL) was heated under reflux for 4 hours. The solution was cooled in ice and a white precipitate formed. The precipitate was filtered off and washed with cold acetonitrile. The solvent was removed from the combined filtrate under reduced pressure to leave an oil which was chromatographed in chloroform/methanol (50:1) yielding a white solid (solid 1). The original solid was dissolved in hot acetonitrile, filtered and the solvent removed under reduced pressure to leave another white solid (solid 2). Both solids (1 and 2) were shown to be 3'-O-acetyl-2,5'-anhydro-E-5-(2-bromovinyl)-2'-deoxyuridine in 61% yield, mp. 220-2$^{o}$C;

$\nu$ max (nujol) 1740, 1630, 1600 $cm^{-1}$;

$^{1}$H nmr [$(CD_3)_2SO$]

$\delta$ 2.02 (3H,s,$CH_3C$=O), 2.6 (2H,m,2'$CH_2$), 4.15 (1H,m,4'CH), 4.54 (2H,m,5'CH), 5.37 (1H,dd. J=2Hz, 7Hz, 3'CH), 6.03 (1H,dd. J=3Hz, 7.5Hz, 1'CH), 6.85 (1H,d,J=14Hz, <u>CH</u> =CHBr), 7.65 (1H,d,J=14Hz, CH=<u>CH</u>Br), 8.0 (1H,s,6-CH).

Analysis

Found: C, 44.09; H, 3.83; N, 7.89% $C_{13}H_{13}BrN_2O_5$
requires: C,43.72; H, 3.67; N, 7.84%.

## ANTIVIRAL ACTIVITY

### In Vitro

#### Method

Vero (African Green Monkey Kidney) cells were grown to confluence in 6 well multidishes, each well being 3.5 cm in diameter. The cells were incubated with Herpes simplex type 1 virus (HFEM strain) and overlaid with 0.5 mL of 0.9% agarose (w/v) in maintenance medium containing the test compound at a range of concentrations from 50 µg/mL in half-log dilution steps. The virus infected cultures were then incubated at 37°C for 6 days before fixing in 4% formaldehyde solution and staining with carbolfuchsin. The dishes were then examined to find that concentration of test compound causing a 50% reduction in the number of virus plaques formed ($PDD_{50}$ value) and the minimum concentration of test compound which killed the cell monolayer, leaving a clear zone devoid of cells and virus plaques (MTD).

#### Results

| Example No. | $PDD_{50}$ (µg/mL) | MTD (µg/mL) |
|---|---|---|
| 1 (b) | 2.8 | > 50 |
| 2 (b) | 13.5 | >100 |

Claims

1. A compound of formula (I):

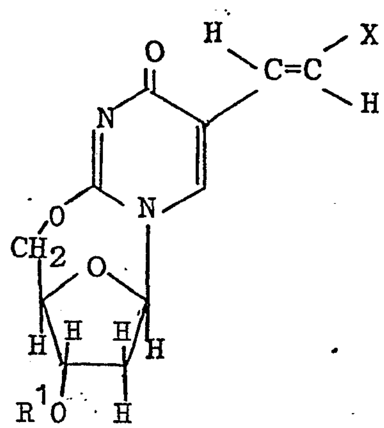

(I)

in which X represents a halogen atom and $R^1$ represents a hydrogen atom or a lower alkanoyl group.

2. A compound according to claim 1, wherein X is a bromine atom.

3. A compound according to claim 1 or 2 wherein $R^1$ represents a lower alkanoyl group.

4. A compound according to claim 1 or 2 wherein $R^1$ represents a hydrogen atom.

5. A compound according to claim 1, selected from 2,5'-anhydro-E-5-(2-bromovinyl)-2'-deoxyuridine and 3'-O-acetyl-2,5'-anhydro-E-5-(2-bromovinyl)-2'-deoxyuridine.

6. A process for producing a compound of formula (I), as defined in claim 1, which comprises heating a compound of formula (II):

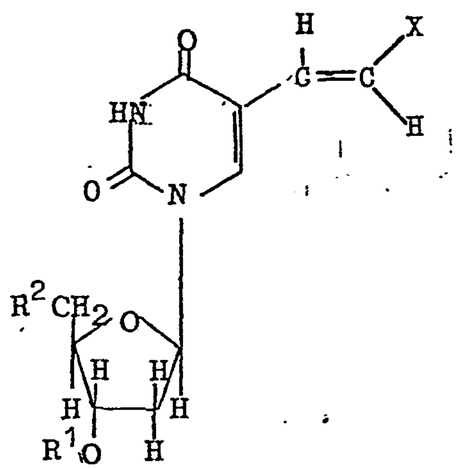

(II)

wherein X represents a halogen atom,

$R^1$ represents a hydrogen atom or a lower alkanoyl group and

$R^2$ represents a leaving group,

in the presence of a strong hindered base.

7. A pharmaceutical composition comprising a pharmaceutically effective amount of a compound of formula (I) as defined in claim 1, together with a pharmaceutically acceptable carrier or excipient.

8. A composition according to claim 7 in unit dose form.

9. A compound of formula (I) as defined in claim 1, for use in the treatment of the human or animal body.

European Patent Office

# EUROPEAN SEARCH REPORT

0060099
Application number

EP 82 30 1121

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | DE-A-2 915 254 (UNIVERSITY OF BIRMINGHAM) *Pages 1-2* | 1,7 | C 07 H 19/06<br>C 07 H 19/08<br>A 61 K 31/70 |
| D | & GB - A - 1 601 020 | | |
| | --- | | |
| Y | CHEMICAL ABSTRACTS, vol. 92, 1980, page 716, no. 198690r, Columbus Ohio USA. K.A.WATANABE et al.:"2,5'-Anhydrouridine and 2,5'-anhydro-5-fluorouridine. One-step conversion of uridine and 5-fluorouridine into their corresponding 2,5'-anhydronucleosides." & NUCL. ACID CHEM. 1978, 1, 343-6 *Abstract* | 1 | |
| | ----- | | **TECHNICAL FIELDS SEARCHED (Int. Cl. 3)** |
| | | | C 07 H 19/00<br>A 61 K 31/00 |

The present search report has been drawn up for all claims

| Place of search | Date of completion of the search | Examiner |
|---|---|---|
| THE HAGUE | 22-04-1982 | VERHULST W. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82